# EUROPÄISCHE PATENTANMELDUNG

(11) **EP 2 213 316 A1**
(43) Veröffentlichungstag der Anmeldung: **04.08.2010**
(21) Anmeldenummer: 10075050.4
(22) Anmeldetag: 02.02.2010
(51) Int. Cl.: A61L 27/54, A61L 29/16, A61L 31/16, A61K 9/00, A61K 33/18, A61K 47/02

(54) **Iod-Beschichtete expandierbare Vorrichtung**

(30) Priorität: 02.02.2009 DE 102009007579
(71) Anmelder: Benjamin Daniel, Orlowski, 51143 Köln (DE)
(72) Erfinder: Benjamin Daniel, Orlowski, 51143 Köln (DE)
(74) Vertreter: Arth, Hans-Lothar

(57) **Zusammenfassung**

Die vorliegende Erfindung betrifft eine expandierbare Vorrichtung zur Aufweitung eines Gefäßlumens, insbesondere einen Katheterballon eines Dilatationskatheters, einen Stent oder die Kombination aus Ballonkatheter und Stent, welche mit elementarem Iod und optional einem Wirkstoff und vorzugsweise einem Träger beschichtet ist.

## Beschreibung

Die vorliegende Erfindung betrifft eine expandierbare Vorrichtung zur Aufweitung eines Gefäßlumens, insbesondere einen Katheterballon eines Dilatationskatheters, einen Stent oder die Kombination aus Ballonkatheter und Stent, welche mit elementarem Iod und optional einem Wirkstoff und vorzugsweise einem Träger beschichtet ist.

Die Implantation von Stents ist heutzutage ein gängiger chirurgischer Eingriff zur Behandlung von Stenosen. Dabei ist noch immer eine sehr häufige Komplikation die sogenannte Restenose (recurrent stenosis), d.h. der Wiederverschluss des Gefäßes. Eine genaue begriffliche Beschreibung der Restenose ist in der Fachliteratur nicht aufzufinden. Die am häufigsten verwendete morphologische Definition der Restenose ist diejenige, die nach erfolgreicher PTA (perkutane transiuminaie Angioplastie) die Restenose als eine Reduktion des Gefäßdurchmessers auf weniger als 50% des normalen festlegt. Hierbei handelt es sich um einen empirisch festgelegten Wert, dessen hämodynamische Bedeutung und Beziehung zur klinischen Symptomatik einer soliden wissenschaftlichen Basis entbehrt. In der Praxis wird häufig die klinische Verschlechterung eines Patienten als Zeichen einer Restenose des vormals behandelten Gefäßabschnitts angesehen.

Die Restenose nach einer Stentimplantation ist eine der Hauptursachen für einen erneuten Krankenhausaufenthalt. Die während der Implantation des Stents verursachten Gefäßverletzungen rufen Entzündungsreaktionen hervor, die für den Heilungsprozess in den ersten sieben Tagen eine entscheidende Rolle spielen. Die hierbei ablaufenden Prozesse sind unter anderem mit der Ausschüttung von Wachstumsfaktoren verbunden, womit eine verstärkte Proliferation der glatten Muskelzellen eingeleitet wird und damit schon kurzfristig zu einer Restenose, einem erneuten Verschluss des Gefäßes aufgrund unkontrollierten Wachstums führen.

Der vorliegenden Erfindung liegt die Beobachtung zugrunde, dass Ausmaß und Auftreten einer Restenose insbesondere durch die erste Woche nach der Stentimplantation bestimmt werden und es daher von besonderer Wichtigkeit ist, Entzündungen während dieser ca. ersten 7 Tage nach erfolgter Stentimplantation möglichst gut zu behandeln.

Aufgabe der vorliegenden Erfindung ist es, eine Möglichkeit der Gefäßaufweitung bereitzustellen, welche die Gefahr der Restenose weitgehend minimiert.

Die Aufgabe wird durch die technische Lehre der unabhängigen Patentansprüche gelöst. Weitere vorteilhafte Ausgestaltungen der Erfindung ergeben sich aus den abhängigen Ansprüchen, der Beschreibung und den Beispielen.

Die vorliegende Erfindung offenbart eine expandierbare Vorrichtung zur Aufweitung eines Gefäßlumens, wobei die expandierbare Vorrichtung mit elementarem Iod und optional einem oder mehreren weiteren Wirkstoffen und optional vorzugsweise einem Träger insbesondere einem polymeren Träger beschichtet ist.

Das elementare Iod (I₂) kann beispielsweise gelöst in einem geeigneten bevorzugt organischen Lösungsmittel oder in Wasser oder in einem organischen Lösungsmittel mit Wasser als Cosolvents gelöst und durch Besprühen, Tauchen, Bestreichen, Pipettieren, oder Vakuumdispersionsverfahren auf eine expandierbare Vorrichtung aufgetragen werden. Als Lösungsmittel eignen sich insbesondere Methanol, Ethanol, Aceton, Toluol, Tetrahydrofuran (THF), Methylenchlorid, Chloroform oder Essigsäureethylester, welche geringe Mengen an Wasser als zusätzliches Lösungsmittel enthalten können.

Als expandierbare Vorrichtung, auf welche das elementare Iod (I₂) mit oder ohne Trägersystem aufgetragen werden kann, kommen insbesondere Stents ohne Beschichtung (bare stents), Stents mit einer vorhandenen Beschichtung, z.B. eine Grundbeschichtung aus Carbon sowie Katheterballons von Dilatationskathetern in Betracht, wobei die Katheterballons ohne aufgesetzten (gecrimpten) Stent beschichtet werden können als auch zusammen mit einem aufgesetzten (gecrimpten Stent). Bei letzterer Ausführungsform wird sowohl die Oberfläche des Stent insbesondere die äußere Oberfläche als auch die Oberfläche des Katheterballons beschichtet. Insbesondere bevorzugt sind jedoch Stents mit einer Trägerbeschichtung, insbesondere einem polymeren Träger oder einem Kontrastmittel, worin das Iod eingelagert ist und woraus es relativ schnell z.B. bei einem Kontrastmittel als Träger oder zeitverzögert, z.B. bei einem polymeren Träger freigesetzt wird.

Die Auftragung des Iods auf die expandierbare Vorrichtung erfolgt, indem das Iod mit der gewünschten Konzentration in einem geeigneten Lösungsmittel gelöst wird und diese Lösung als Tauch- oder Sprühlösung oder Streichlösung oder für die Vakuumabscheidung eingesetzt wird, wobei ein Sprühverfahren bevorzugt ist.

Soll das elementare Iod in einen Träger zur kontrollierten Freisetzung eingebettet werden, so wird der Lösung aus elementarem Iod in einem geeignetem Lösungsmittel noch eine Trägersubstanz, insbesondere ein Polymer oder ein Kontrastmittel zugesetzt. Nach der Aufbringung dieser Lösung auf die expandierbare Vorrichtung verdunstet das Lösungsmittel und das Iod verbleibt eingelagert in dem Träger auf der Oberfläche der expandierbaren Vorrichtung.

Das Iod kann als elementares Iod auf den Stent oder den Katheterballon aufgebracht werden oder zusammen mit einem nicht-polymeren Träger oder einem polymeren Träger. Das Iod kann zur Stabilisierung aber auch zumindest teilweise als lod-Komplex optional zusammen mit einem nicht-polymeren Träger oder einem polymeren Träger auf die Stentoberfläche oder die Katheterballonoberfläche aufgebracht werden.

Als Iod-Komplexe eignen sich Komplexe aus Iod mit Polyvinylpyrrolidon (PVP), oder anderen Vinylpolymeren. Derartige Vinylpolymere werden beispielsweise aus Monovinylverbindungen mit Stickstoffheterocyclen hergestellt, die an einem Ringstickstoffatom mit einer Vinylgruppe substituiert sind. Geeignete Monomere sind vor allem N-Vinyllactame, N-Vinylimidazol und N-Vinylcarbazol. Als N-Vinyllactame kommen 5-, 6- oder 7-gliedrige Lactame in Betracht, die am Ring auch Methyl-, Ethyl- oder Propyl-Substituenten tragen können. Bevorzugte N-Vinyllactame sind N-Vinylpyrrolidon und N-Vinylcaprolactam. Auch Copolymerisate sind geeignet. Weiterhin eignen sich auch Copolymerisate, die neben den Monomeren noch bis zu 80 Gew.-% weitere mono-olefinisch ungesättigte Monomere enthalten, vor allem Vinylester wie Vinylacetat, Vinylpropionat oder Vinylbutyrat, sowie auch Acrylsäure und/oder Methacrylsäure und deren C₁-C₄-Alkylester. Besonders bevorzugte Polymerisate enthalten als Monomere N-Vinylpyrrolidon oder Mischungen aus N-Vinylpyrrolidon und N-Vinylcaprolactam.

Die Monomeren werden in Gegenwart von vernetzend wirkenden Verbindungen polymerisiert. Als vernetzend wirkende Verbindungen kommen vor allem cyclische Amide, die neben einer N-Vinylgruppe eine weitere Vinylgruppe tragen, in Betracht. Geeignete Verbindungen sind beispielsweise cyclische N,N'-Divinylalkylenharnstoffe wie N,N'-Divinylethylenharnstoff, N,N'-Divinyl-propylenharnstoff oder auch N,N'-Divinylimidazolidon oder N-Vinyl-3-ethyliden-pyrrolidon oder N-Vinyl-3-ethyliden-piperidinon.

Als Träger werden bevorzugt Polymere wie beispielsweise biostabile oder bioabbaubare Polymere eingesetzt.

Beispiele für geeignete Träger sind unter anderem:
Polyvalerolactone, Poly-ε-Decalactone, Polylactonsäure, Polyglycolsäure Polylactide, Polyglycolide, Copolymere der Polylactide und Polyglycolide, Poly-ε-caprolacton, Polyhydroxybuttersäure, Polyhydroxybutyrate, Polyhydroxyvalerate, Polyhydroxybutyrate-co-valerate, Poly(1,4-dioxan-2,3-dione), Poly(1,3-dioxan-2-one), Poly-para-dioxanone, Polyanhydride, Polymaleinsäureanhydride, Polyhydroxymethacrylate, Fibrin, Polycyanoacrylate, Polycaprolactondimethylacrylate, Poly-β-Maleinsäure Polycaprolactonbutylacrylate, Multiblockpolymere aus Oligocaprolactondiole und Oligodioxanondiole, Polyetherestermultiblockpolymere aus PEG und Polybutylenterephtalat, Polypivotolactone, Polyglycolsäuretrimethylcarbonate Polycaprolactonglycolide, Poly(y-ethylglutamat), Poly(DTH-Iminocarbonat), Poly(DTE-co-DT-carbonat), Poly(Bisphenol A-iminocarbonat), Polyorthoester, Polyglycolsäuretrimethylcarbonate, Polytrimethylcarbonate Polyiminocarbonate, Poly(N-vinyl)-Pyrrolidon, Polyvinylalkohole, Polyesteramide, glycolierte Polyester, Polyphosphoester, Polyphosphazene, Poly[p-carboxyphenoxy)propan], Polyhydroxypentansäure, Polyanhydride, Polyethylenoxidpropylenoxid, weiche Polyurethane, Polyurethane mit Aminosäurereste im Backbone, Polyetherester wie das Polyethylenoxid, Polyalkenoxalate, Polyorthoester sowie deren Copolymere, Lipide, Carrageenane, Fibrinogen, Stärke, Kollagen, protein-basierende Polymere, Polyaminosäuren, synthetische Polyaminosäuren, Zein, Polyhydroxyalkanoate, Pectinsäure, Actinsäure, Carboxymethylsulfat, Albumin, Hyaluronsäure, Chitosan und seine Derivate, Heparansulfate und seine Derivate, Heparine, Chondroitinsulfat, Dextran, β-Cyclodextrine, Copolymere mit PEG und Polypropylenglycol, Gummi arabicum, Guar, Gelatine, Collagen Collagen-N-Hydroxysuccinimid, Lipide, Phospholipide, Polyacrylsäure, Polyacrylate, Polymethylmethacrylat, Polybutylmethacrylat, Polyacrylamid, Polyacrylonitrile, Polyamide, Polyetheramide, Polyethylenamin, Polyimide, Polycarbonate, Polycarbourethane, Polyvinylketone, Polyvinylhalogenide, Polyvinylidenhalogenide, Polyvinylether, Polyisobutylene, Polyvinylaromaten, Polyvinylester, Polyvinylpyrolidone, Polyoxymethylene, Polytetramethylenoxid, Polyethylen, Polypropylen, Polytetrafluorethylen, Polyurethane, Polyetherurethane, Silicon-Polyetherurethane, Silicon-Polyurethane, Silicon-Polycarbonat-Urethane, Polyolefin-Elastomere, Polyisobutylene, EPDM-Gummis, Fluorosilicone, Carboxymethylchitosane, Polvaryletheretherketone, Polyetheretherketone, Polyethylenterephtalat, Polyvalerate, Carboxymethylcellulose, Cellulose, Rayon, Rayontriacetate, Cellulosenitrate, Celluloseacetate, Hydroxyethylcellulose, Cellulosebutyrate, Celluloseacetatbutyrate, Ethylvinylacetat-copolymere, Polysulfone, Epoxyharze, ABS-Harze, EPDM-Gummis, Silicone wie Polysiloxane, Polydimethylsiloxane, Polyvinylhalogene und Copolymere, Celluloseether, Cellulosetriacetate, Chitosane und Copolymere und/oder Mischungen der vorgenannten Polymere.

Ein weiterer bevorzugter Träger stellt eine Beschichtung aus Parylen dar. Parylen ist die Bezeichnung für vollständig lineare, teilkristalline, unvernetzte aromatische Polymere.

Die verschiedenen Polymere besitzen unterschiedliche Eigenschaften und lassen sich in vier Grundtypen einteilen, nämlich das Parylen C, Parylen D, Parylen N und Parylen F, deren Struktur im Folgenden gezeigt ist:

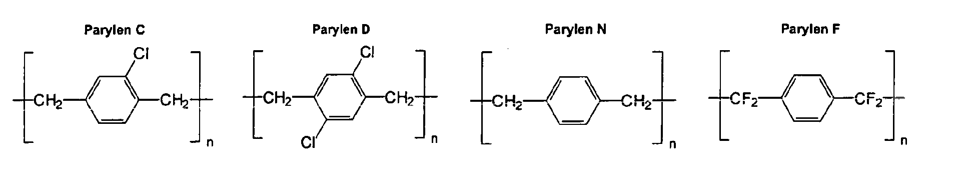

Diese Polymere besitzen sehr gute Eigenschaften hinsichtlich einer lufteinschlussfreien und gleichförmigen Substratbeschichtung, da eine Abscheidung aus der Gasphase erfolgen kann, welche auch erfindungsgemäß Anwendung finden kann. Zudem ist vorteilhaft, dass für die Gasphasenabscheidung auf die zusätzliche Verwendung eines Lösungsmittels verzichtet werden kann.

Die weitere Polymerisation erfolgt dann durch Diffusionsreaktionen auf der Oberfläche der expandierbaren Vorrichtung. Somit wird eine konstante Bedeckung, selbst in Ecken und scharfen Kanten, auf der gesamten zu beschichtenden Oberfläche gewährleistet. Dies ist insbesondere wichtig, wenn die expandierbare Vorrichtung in Form beispielsweise eines Dilatationskatheterballons zusammen mit einem aufgesetzten Stent beschichtet wird.

Das einfachste Monomer der Parylengruppe ist Parylen N (Poly-para-Xylylen). Ferner existieren die zwei chlorierten Polymere Parylen C (Chloropoly-para-Xylylen) und Parylen D (Di-chloro-poly-para-Xylylen). Bei Parylen F (Poly(Tetrafluoro-para-Xylylen)) sind die Methyleneinheiten fluoriert.

Parylen C besitzt den niedrigsten Schmelzpunkt der vorgenannten Parylene von nur 290°C, zeichnet sich durch gute mechanische Eigenschaften und Korrosionsbeständigkeit gegenüber korrosiven Gasen sowie sehr geringe Permeabilität gegenüber Feuchtigkeit aus. Parylen C ist ein biokompatibles Polymer und somit für den Einsatz in physiologischer Umgebung geeignet.

Im Folgenden sind einige Eigenschaften von Parylen C zusammengestellt:

| | |
|---|---|
| maximale Zugspannung | 69Mpa |
| Bruchdehnung | 200% |
| Dichte | 1,289 g/cm³ |
| Reibungskoeffizient (stat.) | 0,29 |
| Reibungskoeffizient (dyn.) | 0,29 |
| Wasseraufnahme | 0,06%/24h |
| Brechungsindex [n] | 1,639 |
| Zersetzungstemperatur | 290°C |
| Thermischer Ausdehnungskoeffizient | 35*10⁻⁶ |
| Durchschlagfestigkeit [Eₘₐₓ] | 2677 V/cm |
| Volumenwiderstand [Rω̅] | 6*10¹⁶ Ohm cm |
| Oberflächenwiderstand [Ro] | 10¹⁵ Ohm cm |

Die Beschichtung der expandierbaren Vorrichtung zur Aufweitung eines Gefäßlumens erfolgt, falls ein Vakuumverfahren benutzt wird, indem man die zu beschichtende Vorrichtung bereitstellt und im Vakuumverfahren bzw. Gasphasenabscheidungsverfahren das Parylen vorzugsweise Parylen C und vorzugsweise ohne Verwendung eines Lösungsmittels auf die expandierbare Vorrichtung aufbringt.

Die Gasphasenabscheidung wird vorzugsweise bei Drücken kleiner 100 Pascal, vorzugsweise kleiner 10 Pa und insbesondere bevorzugt bei ca. 3 Pascal durchgeführt.

Das Iod (I₂) kann zusammen mit dem Parylen C abgeschieden werden, oder das Iod wird vorher mittels beispielsweise Sprühverfahren oder Tauchverfahren auf die expandierbare Vorrichtung aufgebraucht oder nach der Beschichtung der expandierbaren Vorrichtung mit Parylen C mittels beispielsweise Sprühverfahren oder Tauchverfahren auf die Parylen C Schicht aufgetragen.

Ein bevorzugtes Verfahren zur Beschichtung der expandierbaren Vorrichtung ist das sogenannte Ultraschall-Vakuum-Verfahren. Dieses umfast die Schritte:
I) Bereitstellen einer Vakuumkammer,
II) Platzierung der expandierbaren Vorrichtung mittels Haltemittel in der Vakuumkammer,
III) Befüllung einer oder mehrerer Kavitäten innerhalb der Vakuumkammer mit einer Iod-Lösung, elementarem Iod als Feststoff, einer lod-Träger-Lösung oder einer Iod-Lösung und einer Träger-Lösung,
IV) Anlegen von Vakuum an die Vakuumkammer,
V) Erzeugung von Ultraschall in mindestens einer Kavität, welche die zum Aufbringen auf die expandierbare Vorrichtung vorgesehenen Substanzen enthält,
VI) Aufbringen der mittels Ultraschall zerstäubten Substanzen auf die expandierbare Vorrichtung,
VII) Belüftung der Vakuumkammer und Entnahme der beschichteten expandierbaren Vorrichtung.

Bei diesem Verfahren wird eine oder werden mehrere zu beschichtende expandierbare Vorrichtungen in eine Vakuumkammer gegeben, worin sich mindestens eine Kavität mit dem aufzutragenden Polymer oder einer Lösung des aufzutragenden Polymers befindet. Im vorliegenden Fall handelt es sich bei dem Polymer um Parylen C, welches mit oder ohne Lösungsmittel in die Kavität gegeben wird. Es können jedoch auch andere Polymere eingesetzt werden, welche vorzugsweise als Lösung verwendet werden. Für den Fall, dass Parylen C zusammen mit dem Iod aufgebracht werden soll, ist die Verwendung eines Lösungsmittels bevorzugt, da dann eine bessere Zerstäubung stattfinden kann.

Die mindestens eine Kavität in der Vakuumkammer ist derart ausgestaltet, dass darin Ultraschall erzeugt werden kann. Bei dem Beschichtungsverfahren wird nun Vakuum angelegt und ein Unterdruck von mindestens 100 Pa, vorzugsweise mindestens 10 Pascal und insbesondere bevorzugt ca. 3 Pa erzeugt. Nun wird in der mindestens einen Kavität Ultraschall erzeugt und die darin enthaltenen Substanzen werden zerstäubt und scheiden sich auf den zu beschichtenden Gegenständen ab. Teile der zu beschichtenden expandierbaren Vorrichtung, welche nicht beschichtet werden sollen, können beispielsweise durch eine wieder leicht entfernbare Folie abgedeckt werden.

Bevorzugt ist zudem, während der Vakuumbeschichtung einen geringen Inertgasstrom durch die Vorrichtung zu leiten. Ferner kann die Gasphasenbeschichtung mehrmals wiederholt werden, bis die gewünschte Schichtdicke erreicht ist.

Bei einer weiteren bevorzugten Ausführungsform wird die Iod-Wirkstofischicht oder die Trägerschicht enthaltend Iod vorzugsweise mit einer äußeren Barriereschicht versehen. Die äußere Schicht ist bevorzugt eine Polymerschicht, welche vorzugsweise bei der Dilatation der expandierbaren Vorrichtung aufbricht und eine zu frühzeitige unerwünschte Freisetzung des Iods verhindert bzw. stark reduziert.

Diese äußere Schicht vorzugsweise Barriereschicht kann aus einem der oben genannten Polymere oder aus einer Mischung dieser Polymere bestehen und ist bevorzugt hydrophob.

Somit sind erfindungsgemäß auch Mehrschichtsysteme möglich wie beispielsweise ein Zweischichtsystem oder ein Dreischichtsystem, wobei mindestens eine dieser Schichten eine reine Iod-Schicht ist oder mindestens eine dieser Schichten Iod enthält entweder elementar oder als Komplexverbindung oder beides.

Ferner kann die Iod-Schicht und/oder die Iod-enthaltende Schicht und/oder eine weitere Schicht ohne Iod einen antiproliferativen, antiinflammatorischen, antimigrativen, antiphlogistischen, antiangiogenen, cytostatischen, cytotoxischen und/oder antimykotischen Wirkstoff enthalten oder auf die vorgenannten Schichten könnte eine Wirkstoffschicht aus einem antiproliferativen, antiinflammatorischen, antimigrativen, antiphlogistischen, antiangiogenen, cytostatischen, cytotoxischen, antineoplastischen und/oder antimykotischen Wirkstoff aufgebracht werden. Dies bedeutet, dass die expandierbare Vorrichtung mit Iod und mindestens einem weiteren Wirkstoff beschichtet ist, so dass sich auch synergistische Effekte ergeben könnten. Als weiterer Wirkstoff wird ein antiproliferativer, antiinflammatorischer, antimigrativer, antiphlogistischer, antiangiogener, cytostatischer, cytotoxischer und/oder antimykotischer Wirkstoff gewählt.

Beispiele für derartige Wirkstoffe sind:
Abciximab, Acemetacin, Acetylvismion B, Aclarubicin, Ademetionin, Adriamycin, Aescin, Afromoson, Akagerin, Aldesleukin, Amidoron, Aminoglutethemid, Amsacrin, Anakinra, Anastrozol, Anemonin, Anopterin, Antimykotika, Antithrombotika, Apocymarin, Argatroban, Aristolactam-AII, Aristolochsäure, Ascomycin, Asparaginase, Aspirin, Atorvastatin, Auranofin, Azathioprin, Azithromycin, Baccatin, Bafilomycin, Basiliximab, Bendamustin, Benzocain, Berberin, Betulin, Betulinsäure, Bilobol, Bisparthenolidin, Bleomycin, Bombrestatin, Boswellinsäuren und ihre Derivate, Bruceanole A,B und C, Bryophyllin A, Busulfan, Antithrombin, Bivalirudin, Cadherine, Camptothecin, Capecitabin, o-Carbamoylphenoxyessigsäure, Carboplatin, Carmustin, Celecoxib, Cepharantin, Cerivastatin, CETP-Inhibitoren, Chlorambucil, Chloroquinphosphat, Cictoxin, Ciprofloxacin, Cisplatin, Cladribin, Clarithromycin, Colchicin, Concanamycin, Coumadin, C-Type Natriuretic Peptide (CNP), Cudraisoflavon A, Curcumin, Cyclophosphamid, Cyclosporin A, Cytarabin, Dacarbazin, Daclizumab, Dactinomycin, Dapson, Daunorubicin, Diclofenac, 1,11-Dimethoxycanthin-6-on, Docetaxel, Doxorubicin, Dunaimycin, Epirubicin, Epothilone A und B, Erythromycin, Estramustin, Etobosid, Everolimus, Filgrastim, Fluroblastin, Fluvastatin, Fludarabin, Fludarabin-5'-dihydrogenphosphat, Fluorouracil, Folimycin, Fosfestrol, Gemcitabin, Ghalakinosid, Ginkgol, Ginkgolsäure, Glykosid 1a, 4-Hydroxyoxycyclophosphamid, Idarubicin, Ifosfamid, Josamycin, Lapachol, Lomustin, Lovastatin, Melphalan, Midecamycin, Mitoxantron, Nimustin, Pitavastatin, Pravastatin, Procarbazin, Mitomycin, Methotrexat, Mercaptopurin, Thioguanin, Oxaliplatin, Irinotecan, Topotecan, Hydroxycarbamid, Miltefosin, Pentostatin, Pegasparase, Exemestan, Letrozol, Formestan, SMC-Proliferation-Inhibitor-2w, Mitoxanthrone, Mycophenolatmofetil, c-myc-Antisense, β-myc-Antisense, β-Lapachon,Podophyllotoxin, Podophyllsäure-2-ethylhydrazid, Molgramostim (rhuGM-CSF), Peginterferon α-2b, Lanograstim (r-HuG-CSF), Macrogol, Selectin (Cytokinantagonist), Cytokininhibitoren, COX-2-Inhibitor, NFkB, Angiopeptin, monoklonale Antikörper, die die Muskelzellproliferation hemmen, bFGF-Antagonisten, Probucol, Prostaglandine, 1-Hydroxy-11-Methoxycanthin-6-on, Scopolectin, NO-Donoren wie Pentaerythrityltetranitrat und Syndnoeimine, S-Nitrosoderivate, Tamoxifen, Staurosporin, β-Estradiol, α-Estradiol, Estriol, Estron, Ethinylestradiol, Medroxyprogesteron, Estradiolcypionate, Estradiolbenzoate, Tranilast, Kamebakaurin und andere Terpenoide, die in der Krebstherapie eingesetzt werden, Verapamil, Tyrosin-Kinase-Inhibitoren (Tyrphostine), Paclitaxel und dessen Derivate wie 6-α-Hydroxy-Paclitaxel, Taxotere, Kohlensuboxids (MCS) und dessen macrocyclische Oligomere, Mofebutazon, Lonazolac, Lidocain, Ketoprofen, Mefenaminsäure, Piroxicam, Meloxicam, Penicillamin, Hydroxychloroquin, Natriumaurothiomalat, Oxaceprol, β-Sitosterin, Myrtecain, Polidocanol, Nonivamid, Levomenthol, Ellipticin, D-24851 (Calbiochem), Colcemid, Cytochalasin A-E, Indanocine, Nocadazole, S 100 Protein, Bacitracin, Vitronectin-Rezeptor Antagonisten, Azelastin, Guanidylcyclase-Stimulator Gewebsinhibitor der Metallproteinase-1 und 2, freie Nukleinsäuren, Nukleinsäuren in Virenüberträger inkorporiert, DNA- und RNA-Fragmente, Plaminogen-Aktivator Inhibitor-1, Plasminogen-Aktivator Inhibitor-2, Antisense Oligonucleotide, VEGF-Inhibitoren, IGF-1, Wirkstoffe aus der Gruppe der Antibiotika wie Cefadroxil, Cefazolin, Cefaclor, Cefotixin Tobramycin, Gentamycin, Penicilline wie Dicloxacillin, Oxacillin, Sulfonamide, Metronidazol, Enoxoparin, desulfatiertes und N-reacetyliertes Heparin (Hemoparin®), Gewebe-Plasminogen-Aktivator, GpIIb/IIIa-Plättchenmembranrezeptor, Faktor Xₐ-Inhibitor Antikörper, Heparin, Hirudin, r-Hirudin, PPACK, Protamin, Prourokinase, Streptokinase, Warfarin, Urokinase, Vasodilatoren wie Dipyramidol, Trapidil, Nitroprusside, PDGF-Antagonisten wie Triazolopyrimidin und Seramin, ACE-Inhibitoren wie Captopril, Cilazapril, Lisinopril, Enalapril, Losartan, Thioproteaseinhibitoren, Prostacyclin, Vapiprost, Interferon α, β und γ, Histaminantagonisten, Serotoninblocker, Apoptoseinhibitoren, Apoptoseregulatoren wie p65, NF-kB oder Bcl-xL-Antisense-Oligonukleotiden, Halofuginon, Nifedipin, Tocopherol Tranilast, Molsidomin, Teepolyphenole, Epicatechingallat, Epigallocatechingallat, Leflunomid, Etanercept, Sulfasalazin, Etoposid, Dicloxacyllin, Tetracyclin, Triamcinolon, Mutamycin, Procainimid, Retinolsäure, Quinidin, Disopyrimid, Flecainid, Propafenon, Sotolol, natürliche und synthetisch hergestellte Steroide wie Inotodiol, Maquirosid A, Ghalakinosid, Mansonin, Streblosid, Hydrocortison, Betamethason, Dexamethason, nichtsteroidale Substanzen (NSAIDS) wie Fenoporfen, Ibuprofen, Indomethacin, Naproxen, Phenylbutazon und andere antivirale Agentien wie Acyclovir, Ganciclovir und Zidovudin, Clotrimazol, Flucytosin, Griseofulvin, Ketoconazol, Miconazol, Nystatin, Terbinafin, antiprozoale Agentien wie Chloroquin, Mefloquin, Quinin, des weiteren natürliche Terpenoide wie Hippocaesculin, Barringtogenol-C21-angelat, 14-Dehydroagrostistachin, Agroskerin, Agrostistachin, 17-Hydroxyagrostistachin, Ovatodiolide, 4,7-Oxycydoanisomelsäure, Baccharinoide B1, B2, B3 und B7, Tubeimosid, Bruceantinoside C, Yadanzioside N, und P, Isodeoxyelephantopin, Tomenphantopin A und B, Coronarin A,B,C und D, Ursolsäure, Hyptatsäure A, Iso-Iridogermanal. Maytenfoliol, Effusantin A, Excisanin A und B, Longikaurin B, Sculponeatin C, Kamebaunin, Leukamenin A und B, 13,18-Dehydro-6-alpha-Senecioyloxychaparrin, Taxamairin A und B, Regenilol, Triptolid, des weiteren Cymarin, Hydroxyanopterin, Protoanemonin, Cheliburinchlorid, Sinococulin A und B, Dihydronitidin, Nitidinchlorid, 12-beta-Hydroxypregnadien 3,20-dion, Helenalin, Indicin, Indicin-N-oxid, Lasiocarpin, Inotodiol, , Podophyllotoxin, Justicidin A und B, Larreatin, Malloterin, Mallotochromanol, Isobutyrylmallotochromanol, Maquirosid A, Marchantin A, Maytansin, Lycoridicin, Margetin, Pancratistatin, Liriodenin, Bispsrthenolidin, Oxoushinsunin, Periplocosid A, Ursolsäure, Deoxypsorospermin, Psycorubin, Ricin A, Sanguinarin, Manwuweizsäure, Methylsorbifolin, Sphatheliachromen, Stizophyllin, Mansonin, Streblosid, Dihydrousambaraensin, Hydroxyusambarin, Strychnopentamin, Strychnophylin, Usambarin, Usambarensin, Liriodenin, Oxoushinsunin, Daphnoretin, Lariciresinol, Methoxylariciresinol, Syringaresinol, Sirolimus (Rapamycin), Somatostatin, Tacrolimus, Roxithromycin, Troleandomycin, Simvastatin, Rosuvastatin, Vinblastin, Vincristin, Vindesin, Teniposid, Vinorelbin, Tropfosfamid, Treosu!fan, Tremozolomid, Thiotepa, Tretinoin, Spiramycin, Umbelliferon, Desacetylvismion A, Vismion A und B, Zeorin.

Der Wirkstoff kann in reiner Form oder zusammen mit einem Träger vorzugsweise einem polymeren Träger aufgebracht werden. Für die Aufbringung werden bevorzugt Tauch-, Sprüh-, Streich- oder Vakuumverfahren eingesetzt.

Als Träger können jedoch auch nicht-polymere Substanzen eingesetzt werden wie beispielsweise Kontrastmittel. Werden derartige Trägersysteme verwendet, so wird vorzugsweise eine Lösung aus elementarem Iod, Kontrastmittel und Lösungsmittel hergestellt, welche als Sprühlösung oder Tauchlösung zur Beschichtung der expandierbaren Vorrichtungen eingesetzt wird.

Bei den Kontrastmitteln handelt es sich zumeist um Stoffe, welche Barium, Iod, Mangan, Eisen, Lanthan, Cer, Praseodym, Neodym, Samarium, Europium, Gadolinium, Terbium, Dysprosium, Holmium, Erbium, Thulium, Ytterbium und/oder Lutetium enthalten.

Als Kontrastmittel können übliche Kontrastmittel für Röntgenaufnahmen, Computertomographie (CT), Kernspintomographie oder Magnetresonanztomographie (MRT) eingesetzt werden.

Prinzipiell zu unterscheiden sind Kontrastmittel, die bei Röntgenuntersuchungen zum Einsatz kommen (Röntgenkontrastmittel), und die, die bei magnetresonanztomographischen Untersuchungen eingesetzt werden (MR-Kontrastmittel), wobei die Röntgenkontrastmittel wie z.B. Jod-Lipiodol^{®} bevorzugt sind.

Im Falle von Röntgenkontrastmitteln handelt es sich um Substanzen, die entweder zu einer vermehrten Absorption einfallender Röntgenstrahlen gegenüber der umgebenden Struktur führen (sog. positive Kontrastmittel) oder einfallende Röntgenstrahlen vermehrt ungehindert durchlassen (sog. negative Kontrastmittel).

Des Weiteren sind iodhaltige Kontrastmittel bevorzugt, welche bei der Gefäßdarstellung (Angiographie und Phlebographie) und bei der CT (Computertomographie) verwendet werden.

Insbesondere bevorzugt sind Kontrastmittel mit einem 1,3,5-Triiodbenzolkern, nephrotrope niederosmolare Röntgenkontrastmittel, Amidotrizoesäure, lothalaminsäure, lotrolan, lopamidol, lodoxaminsäure, Diatrizoesäure, lomeprol, lopromid, Desmethoxyacetyl-lopromid (DAMI) oder 5-Amino-2,4,6-triiodphthalsäure-(2,3-dihydroxypropyl)-amid (ATH).

Eine weitere Klasse von bevorzugten Kontrastmitteln stellen die paramagnetischen Kontrastmittel dar, welche zumeist ein Lanthanoid enthalten.

Zu den paramagnetischen Substanzen, die über ungepaarte Elektronen verfügen, zählt z.B. das Gadolinium (Gd³⁺), das insgesamt sieben ungepaarte Elektronen besitzt. Des Weiteren gehören zu dieser Gruppe das Europium (Eu²⁺, Eu³⁺), Dysprosium (Dy³⁺) und Holmium (Ho³⁺). Diese Lanthanoide können auch in chelatisierter Form unter Verwendung von beispielsweise Hämoglobin, Chlorophyll, Polyazasäuren, Polycarbonsäuren und insbesondere EDTA, DTPA sowie DOTA als Chelatbildner eingesetzt werden. Beispiele für Gadolinium-haltige Kontrastmittel sind Gadolinium-Diethylentriaminpentaessigsäure, Gadopentetsäure (GaDPTA), Gadodiamid, Meglumin-Gadoterat oder Gadoteridol.

Eine weitere bevorzugte Ausführungsform betrifft eine Gefäßstütze insbesondere einen Stent als expandierbare Vorrichtung, wobei die vorzugsweise metallische Oberfläche der Gefäßstütze mit einer Schicht aus Carbon oder Siliziumcarbid oder pyrolytischem Carbon oder diamantenähnlichem Kohlenstoff (diamond-like carbon: DLC) versehen wird bzw. überdeckt wird. Diese Beschichtung mit Carbon, DLC oder Siliziumcarbid erfolgt mittels Ionenimplantation oder Plasmaverfahren und generiert eine Barriereschicht, welche den Austritt von Ionen aus dem Stentmaterial verhindert, welche zu allergischen Reaktionen führen können.

Ferner ist eine insbesondere bevorzugte Ausführungsform eine Gefäßstütze, insbesondere ein Stent, welche mit einer porösen Carbonschicht versehen ist. Dazu wird eine Gefäßstütze zuerst mit einer porösen Kohlenstoffschicht versehen und danach wird in die Poren dieser Kohlenstoffschicht das Iod eingebracht. Das Iod wird dazu in einem vorzugsweise flüchtigen Lösungsmittel gelöst und die Gefäßstütze wird in diese Lösung mehrmals getaucht und nach jedem Tauchvorgang getrocknet oder mehrmals mit dieser Lösung besprüht. Das Iod sammelt sich bevorzugt in den Poren der Kohlenstoffschicht an, wenn das Lösungsmittel verdunstet. Durch die Ansammlung in den Poren ist das Iod auch geschützt gegen Elution während des Einführens des Katheters.

Ferner kann der mit Iod ohne Träger beschichtete Stent mit einer Barriereschicht und/oder einer äußeren hämokompatiblen Schicht versehen werden, wobei das Iod durch diese Barriereschicht und/oder diese äußere hämokompatible Schicht diffundieren und in das umliegende Gewebe gelangen kann. Als Barriereschicht und/oder äußere hämokompatible Schicht werden vorzugsweise die oben genannten Polymere eingesetzt, welche auch als Träger dienen können.

Bei den expandierbaren Vorrichtungen handelt es sich insbesondere um eine Gefäßstütze vorzugsweise für Koronargefäße, einen Stent oder einen Ballon eines Katheters insbesondere eines Dilatationskatheters, wobei es auch möglich ist, dass der Ballon mit aufgesetztem (gecrimptem) Stent zusammen beschichtet wird. Hier werden also Ballon mit aufgesetztem Stent als eine Einheit beschichtet. Dabei können diverse herkömmliche Ballons sowie Stents eingesetzt werden. Der verwendete Stent bzw. die Gefäßstütze kann aus medizinischem Edelstahl, Titan, Cobalt-Chrom, Chrom, Vanadium, Magnesium, Yttrium, Wolfram, Molybdän, Gold, Nitinol, Legierungen, Teflon oder anderen Kunststoffen bestehen, wobei metallische Stents bevorzugt sind. Der Stent kann auch bioresorbierbar sein, d.h. sich nach einer gewissen Zeit vollständig auflösen wie beispielsweise Magnesiumstents oder Stents aus Polyhydroxybutyrat. Ferner kann der aufgesetzte Stent bereits beschichtet sein, wobei natürlich auch unbeschichtete Stents sich auf dem Dilatationsballon befinden können. Auch Stents mit einer biostabilen, bioabbaubaren und/oder hämokompatiblen Schicht können eingesetzt werden.

Handelt es sich bei der expandierbaren Vorrichtung um einen Ballon eines Katheters vorzugsweise eines Dilatationskatheters dann besteht der Ballon vorzugsweise aus einem polymeren Material wie z.B. Polyester, Polyethylen, Polysulfon, wobei jedoch Polyamid bevorzugt ist.

Bei der Implantation ergibt sich nun der Vorteil, dass bei der Expansion zumindest für kurze Zeit die Oberfläche des Ballons mit oder ohne Stent gegen die Gefäßwand gedrückt wird. Diese Expansion kann gleichzeitig zwei Aufgaben erfüllen. Wird ein Stent verwendet, so wird dieser expandiert und somit in seine Endform und Lage gebracht und zum anderen wird das Iod eventuell zusammen mit weiteren Wirkstoffen von der Ballonoberfläche auf die Gefäßwand übertragen. Das von der Ballonoberfläche auf die Gefäßinnenwand bei der Dilatation übertragene Iod optional zusammen mit einem weiteren Wirkstoff liegt in hoher Konzentration vor und wird relativ schnell verbraucht oder von dem Gewebe und den Zellen aufgenommen.

Diese Vorgehensweise führt jedoch dazu, dass die gesamten Gefaßinnenwand für die erste Woche nach Dilatation bzw. nach Stentimplantation mit einer ausreichenden Menge an Iod eventuell zusammen mit einem weiteren Wirkstoff versorgt wird. Als weitere Wirkstoffe eignen such insbesondere Paclitaxel und Rapamycin.

Ferner ist für die Barriereschicht die Verwendung eines Polymeren bevorzugt, welches eine ausreichende Stabilität gegen Abrieb während der Einführung des

Dilatationskatheters aufweist, zum Zeitpunkt der Expansion des Ballons jedoch in der Lage ist, die Trägerschicht mit dem Iod-Wirkstoff-Gemisch oder die reine Iodschicht auf die Gefäßinnenwand zu übertragen.

Die expandierbare Vorrichtung umfasst vorzugsweise einen Ballon in nichtexpandiertem Zustand. Ferner kann das System noch einen Katheter und/oder Stent umfassen, so dass die Möglichkeit besteht, ein erfindungsgemäß beschichtetes System aus Ballon mit aufgesetztem Stent und ohne Katheter oder ein erfindungsgemäß beschichtetes System aus Ballon mit Katheter und aufgesetztem Stent bereitzustellen und zu vermarkten.

Bei der erfindungsgemäßen expandierbaren Vorrichtung handelt es sich bevorzugt um Gefäßstützen für kanalartige Strukturen und insbesondere um Stents für Blutgefäße, Harnwege, Atemwege, Gallenwege, das kardiovaskulare System oder den Verdauungstrakt.

### Beispiele

### Beispiel 1:

Ein kommerziell erhältlicher Dilatationskatheter mit expandierbarem Ballon wird derart vorbereitet, dass nur der Ballon frei bleibt und der restliche Teil des Katheters durch eine wieder leicht entfernbare Plastikfolie abgedeckt wird.

Die so vorbereitete expandierbare Vorrichtung wird in eine Vakuumkammer gegeben, worin sich eine Lösung aus Parylen C und Iod befindet.

Nun wird Vakuum angelegt und der Ballon wird bei ca. 3 Pa im Vakuum beschichtet.

Danach wird die expandierbare Vorrichtung entnommen und die Plastikfolie entfernt.

### Beispiel 2:

Auf die gemäß Beispiel 1 beschichtete expandierbare Vorrichtung wird mittels Sprühverfahren eine Barriereschicht aus einem Polylactid aufgetragen. Der Sprühvorgang wird mehrmals wiederholt und durch Trocknungsschritte unterbrochen.

### Beispiel 3:

Der Ballon eines PTCA-Katheters mit aufgesetztem Stent aus Cobalt-Chrom wird im Sprühverfahren mit einer methanolischen Lösung aus Polyester und elementarem Iod insgesamt 6 Mal besprüht und nach jedem Sprühschritt getrocknet.

### Beispiel 4:

Der Ballon eines PTCA-Katheters ohne aufgesetzten (gecrimpten) Stent wird im Tauchverfahren mit einer Lösung aus Polyester und Iod (20 µg / ml) in THF insgesamt 3 Mal beschichtet und nach jedem Tauchschritt getrocknet.

### Beispiel 5:

Ein Nitinol-Stent wird mittels Sprühverfahren mit einer Lösung aus Iod (80 µg / ml) und einem Polyglykolid in THF mittels Sprühverfahren beschichtet. Bei der Beschichtung wird der Stent entlang seiner Längsachse gedreht und mehrmals mit der vorgenannten Lösung besprüht und nach jedem Sprühvorgang wird der Stent getrocknet. Die Sprühzyklen werden so oft wiederholt bis sich ca. 5 µg elementares Iod auf dem ca. 1,8 cm langen Stent befinden.

### Beispiel 6:

Ein Katheterballon mit gecrimptem Stent wird mittels Sprühverfahren mit einer Lösung aus Iod und Dextran in Chloroform mehrmals besprüht und nach jedem Sprühvorgang getrocknet. Die Sprüh- und Trocknungsvorgänge werden so oft wiederholt, bis sich ca. 2,2 µg Iod pro cm² Ballonoberfläche befinden.

### Beispiel 7:

Ein Nitinol-Stent wird mittels Sprühverfahren mit einer Lösung aus Iod (100 µg / ml) und einem Polyether in THF mittels Sprühverfahren beschichtet. Bei der Beschichtung wird der Stent entlang seiner Längsachse gedreht und mehrmals mit der vorgenannten Lösung besprüht und nach jedem Sprühvorgang wird der Stent getrocknet. Die Sprühzyklen werden so oft wiederholt bis sich ca. 30 µg Iod auf dem ca. 1,4 cm langen Stent befinden.

### Beispiel 8:

Ein kommerziell erhältlicher Katheter mit einem Katheterballon aus Polyamid wird mit einer Lösung aus elementarem Iod (60 µg / ml) und Paclitaxel in DMSO mittels Tauchverfahren beschichtet.

Die Beschichtung wird nach jedem Tauchvorgang getrocknet und der Tauchvorgang wird zweimal wiederholt. Man erhält eine gleichmäßige Beschichtung der gesamten Oberfläche inklusive der Falten des Katheterballons.

Ein kommerziell erhältlicher Vanadium-Stent wird mit einer polymeren Beschichtung aus einem Polyurethan enthaltend elementares Iod vorzugsweise beschichtet.

Der beschichtete Stent wird nun auf den beschichteten Katheterballon gecrimpt.

### Beispiel 9:

Ein Nitinol-Stent wird mit einer porösen Kohlenstoffschicht versehen. Mittels Sprühverfahren mit einer Lösung aus Iod (100 µg / ml) werden die Poren mit Iod beschichtet. Bei der Beschichtung wird der Stent entlang seiner Längsachse gedreht und mehrmals mit der vorgenannten Lösung besprüht und nach jedem Sprühvorgang wird der Stent getrocknet. Die Sprühzyklen werden so oft wiederholt bis sich ca. 30 µg Iod auf dem ca. 1,4 cm langen Stent befinden.

### Beispiel 10:

Ein Katheterballon mit gecrimptem Stent wird mittels Sprühverfahren mit einer Lösung aus lod-PVP in Ethanol mehrmals besprüht und nach jedem Sprühvorgang getrocknet. Die Sprüh- und Trocknungsvorgänge werden so oft wiederholt, bis sich ca. 10 µg lod-PVP pro cm² Ballonoberfläche befinden.

## Patentansprüche

1. Expandierbare Vorrichtung zur Aufweitung eines Gefäßlumens, wobei die expandierbare Vorrichtung mit elementarem Iod oder einer lod-Komplexverbindung beschichtet ist.

2. Expandierbare Vorrichtung nach Anspruch 1, wobei die expandierbare Vorrichtung mit Iod und Paclitaxel oder mit Iod und Rapamycin beschichtet ist.

3. Expandierbare Vorrichtung nach Anspruch 1 oder 2, wobei die Beschichtung des weiteren einen Träger umfasst.

4. Expandierbare Vorrichtung nach Anspruch 3, wobei der Träger ausgewählt wird aus der Gruppe umfassend:
Parylen C, Parylen D, Parylen N, Parylen F, Polyvalerolactone, Poly-ε-Decalactone, Polylactonsäure, Polyglycolsäure Polylactide, Polyglycolide, Copolymere der Polylactide und Polyglycolide, Poly-ε-caprolacton, Polyhydroxybuttersäure, Polyhydroxybutyrate, Polyhydroxyvalerate, Polyhydroxybutyrate-co-valerate, Poly(1,4-dioxan-2,3-dione), Poly(1,3-dioxan-2-one), Poly-para-dioxanone, Polyanhydride, Polymaleinsäureanhydride, Polyhydroxymethacrylate, Fibrin, Polycyanoacrylate, Polycaprolactondimethylacrylate, Poly-β-Maleinsäure Polycaprolactonbutylacrylate, Multiblockpolymere aus Oligocaprolactondiole und Oligodioxanondiole, Polyetherestermultiblockpolymere aus PEG und Polybutylenterephtalat, Polypivotolactone, Polyglycolsäuretrimethylcarbonate Polycaprolactonglycolide, Poly(y-ethylglutamat), Poly(DTH-Iminocarbonat), Poly(DTE-co-DT-carbonat), Poly(Bisphenol A-iminocarbonat), Polyorthoester, Polyglycolsäuretrimethylcarbonate, Polytrimethylcarbonate Polyiminocarbonate, Poly(N-vinyl)-Pyrrolidon, Polyvinylalkohole, Polyesteramide, glycolierte Polyester, Polyphosphoester, Polyphosphazene, Poly[p-carboxyphenoxy)propan], Polyhydroxypentansäure, Polyanhydride, Polyethylenoxidpropylenoxid, weiche Polyurethane, Polyurethane mit Aminosäurereste im Backbone, Polyetherester, Polyethylenoxid, Polyalkenoxalate, Polyorthoester sowie deren Copolymere, Lipide, Carrageenane, Fibrinogen, Stärke, Kollagen, protein-basierende Polymere, Polyaminosäuren, synthetische Polyaminosäuren, Zein, Polyhydroxyalkanoate, Pectinsäure, Actinsäure, Carboxymethylsulfat, Albumin, Hyaluronsäure, Chitosan und seine Derivate, Heparansulfate und seine Derivate, Heparine, Chondroitinsulfat, Dextran, β-Cyclodextrine, Copolymere mit PEG und Polypropylenglycol, Gummi arabicum, Guar, Gelatine, Collagen Collagen-N-Hydroxysuccinimid, Lipide, Phospholipide, Polyacrylsäure, Polyacrylate, Polymethylmethacrylat, Polybutylmethacrylat, Polyacrylamid, Polyacrylonitrile, Polyamide, Polyetheramide, Polyethylenamin, Polyimide, Polycarbonate, Polycarbourethane, Polyvinylketone, Polyvinylhalogenide, Polyvinylidenhalogenide, Polyvinylether, Polyisobutylene, Polyvinylaromaten, Polyvinylester, Polyvinylpyrollidone, Polyoxymethylene, Polytetramethylenoxid, Polyethylen, Polypropylen, Polytetrafluorethylen, Polyurethane, Polyetherurethane, Silicon-Polyetherurethane, Silicon-Polyurethane, Silicon-Polycarbonat-Urethane, Polyolefin-Elastomere, Polyisobutylene, EPDM-Gummis, Fluorosilicone, Carboxymethylchitosane, Polyaryletheretherketone, Polyetheretherketone, Polyethylenterephtalat, Polyvalerate, Carboxymethylcellulose, Cellulose, Rayon, Rayontriacetate, Cellulosenitrate, Celluloseacetate, Hydroxyethylcellulose, Cellulosebutyrate, Celluloseacetatbutyrate, Ethylvinylacetat-copolymere, Polysulfone, Epoxyharze, ABS-Harze, EPDM-Gummis, Silicone, Polysiloxane, Polydimethylsiloxane, Polyvinylhalogene und Copolymere, Celluloseether, Cellulosetriacetate, Chitosane und Copolymere und/oder Mischungen der vorgenannten Polymere.

5. Expandierbare Vorrichtung nach Anspruch 3, wobei der Träger ausgewählt wird aus der Gruppe umfassend:
Kontrastmittel, Röntgenkontrastmittel und iodhaltige Kontrastmittel.

6. Expandierbare Vorrichtung nach Anspruch 1, wobei die Iod-Komplexverbindung ausgewählt wird aus der Gruppe umfassend:
Iod-Komplexe mit Vinylpolymeren, mit Polyvinylpyrrolidon oder mit Vinylpolymeren oder Vinylcopolymerisaten aus N-Vinyllactamen, N-Vinylimidazol, N-Vinylcarbazol, N-Vinylpyrrolidon, N-Vinylcaprolactamen, Vinylestern, Vinylacetat, Vinylpropionat, Vinylbutyrat, Acrylsäure, Acrylsäureestern, Methacrylsäure und Methacrylsäureestern.

7. Expandierbare Vorrichtung nach einem der vorherigen Ansprüche, des weiteren umfassend mindestens einen antiproliferativen, antiinflammatorischen, antimigrativen, antiphlogistischen, antiangiogenen, cytostatischen, cytotoxischen, antineoplastischen und/oder antimykotischen Wirkstoff.

8. Expandierbare Vorrichtung nach Anspruch 7, wobei der mindestens eine antiproliferative, antiinflammatorische, antimigrative, antiphlogistische, antiangiogene, cytostatische, cytotoxische, antineoplastischen und/oder antimykotische Wirkstoff aus der Gruppe ausgewählt wird umfassend:
Abciximab, Acemetacin, Acetylvismion B, Aclarubicin, Ademetionin, Adriamycin, Aescin, Afromoson, Akagerin, Aldesleukin, Amidoron, Aminoglutethemid, Amsacrin, Anakinra, Anastrozol, Anemonin, Anopterin, Antimykotika, Antithrombotika, Apocymarin, Argatroban, Aristolactam-All, Aristolochsäure, Ascomycin, Asparaginase, Aspirin, Atorvastatin, Auranofin, Azathioprin, Azithromycin, Baccatin, Bafilomycin, Basiliximab, Bendamustin, Benzocain, Berberin, Betulin, Betulinsäure, Bilobol, Bisparthenolidin, Bleomycin, Bombrestatin, Boswellinsäuren und ihre Derivate, Bruceanole A,B und C,
Bryophyllin A, Busulfan, Antithrombin, Bivalirudin, Cadherine, Camptothecin, Capecitabin, o-Carbamoylphenoxyessigsäure, Carboplatin, Carmustin, Celecoxib, Cepharantin, Cerivastatin, CETP-Inhibitoren, Chlorambucil, Chloroquinphosphat, Cictoxin, Ciprofloxacin, Cisplatin, Cladribin, Clarithromycin, Colchicin, Concanamycin, Coumadin, C-Type Natriuretic Peptide (CNP), Cudraisoflavon A, Curcumin, Cyclophosphamid, Cyclosporin A, Cytarabin, Dacarbazin, Daclizumab, Dactinomycin, Dapson, Daunorubicin, Diclofenac, 1,11-Dimethoxycanthin-6-on, Docetaxel, Doxorubicin, Dunaimycin, Epirubicin, Epothilone A und B, Erythromycin, Estramustin, Etobosid, Everolimus, Filgrastim, Fluroblastin, Fluvastatin, Fludarabin, Fludarabin-5'-dihydrogenphosphat, Fluorouracil, Folimycin, Fosfestrol, Gemcitabin, Ghalakinosid, Ginkgol, Ginkgolsäure, Glykosid 1a, 4-Hydroxyoxycyclophosphamid, Idarubicin, Ifosfamid, Josamycin, Lapachol, Lomustin, Lovastatin, Melphalan, Midecamycin, Mitoxantron, Nimustin, Pitavastatin, Pravastatin, Procarbazin, Mitomycin, Methotrexat, Mercaptopurin, Thioguanin, Oxaliplatin, Irinotecan, Topotecan, Hydroxycarbamid, Miltefosin, Pentostatin, Pegasparase, Exemestan, Letrozol, Formestan, Mitoxanthrone, Mycophenolatmofetil, β-Lapachon, Podophyllotoxin, Podophyllsäure-2-ethylhydrazid, Molgramostim (rhuGM-CSF), Peginterferon α-2b, Lanograstim (r-HuG-CSF), Macrogol, Selectin (Cytokinantagonist), Cytokininhibitoren, COX-2-Inhibitor, Angiopeptin, monoklonale Antikörper, die die Muskelzellproliferation hemmen, bFGF-Antagonisten, Probucol, Prostaglandine, 1-Hydroxy-11-Methoxycanthin-6-on, Scopolectin, NO-Donoren, Pentaerythrityltetranitrat und
Syndnoeimine, S-Nitrosoderivate, Tamoxifen, Staurosporin, β-Estradiol, α-Estradiol, Estriol, Estron, Ethinylestradiol, Medroxyprogesteron, Estradiolcypionate, Estradiolbenzoate, Tranilast, Kamebakaurin und andere Terpenoide, die in der Krebstherapie eingesetzt werden, Verapamil, Tyrosin-Kinase-Inhibitoren (Tyrphostine), Paclitaxel und dessen Derivate, 6-α-Hydroxy-Paclitaxel, Taxotere, Mofebutazon, Lonazolac, Lidocain, Ketoprofen, Mefenaminsäure, Piroxicam, Meloxicam. Penicillamin, Hydroxychioroquin, Natriumaurothiomalat, Oxaceprol, β-Sitosterin, Myrtecain, Polidocanol, Nonivamid, Levomenthol, Ellipticin, D-24851 (Calbiochem), Colcemid, Cytochalasin A-E, Indanocine, Nocadazole, Bacitracin, Vitronectin-Rezeptor Antagonisten, Azelastin, Guanidylcyclase-Stimulator Gewebsinhibitor der Metallproteinase-1 und 2, freie Nukleinsäuren, Nukleinsäuren in Virenüberträger inkorporiert, DNA- und RNA-Fragmente, Plaminogen-Aktivator Inhibitor-1, Plasminogen-Aktivator Inhibitor-2, Antisense Oligonucleotide, VEGF-Inhibitoren, IGF-1, Wirkstoffe aus der Gruppe der Antibiotika, Cefadroxil, Cefazolin, Cefaclor, Cefotixin Tobramycin, Gentamycin, Penicilline, Dicloxacillin, Oxacillin, Sulfonamide, Metronidazol, Enoxoparin, Heparin, Hirudin, PPACK, Protamin, Prourokinase, Streptokinase, Warfarin, Urokinase, Vasodilatoren, Dipyramidol, Trapidil, Nitroprusside, PDGF-Antagonisten, Triazolopyrimidin, Seramin, ACE-Inhibitoren, Captopril, Cilazapril, Lisinopril, Enalapril, Losartan, Thioproteaseinhibitoren, Prostacyclin, Vapiprost, Interferon α, β und γ, Histaminantagonisten, Serotoninblocker, Apoptoseinhibitoren, Apoptoseregulatoren, Halofuginon, Nifedipin, Tocopherol Tranilast, Molsidomin, Teepolyphenole, Epicatechingallat, Epigallocatechingallat, Leflunomid, Etanercept, Sulfasalazin, Etoposid, Dicloxacyllin, Tetracyclin, Triamcinolon, Mutamycin, Procainimid, Retinolsäure, Quinidin, Disopyrimid, Flecainid, Propafenon, Sotolol, natürliche und synthetisch hergestellte Steroide, Inotodiol, Maquirosid A, Ghalakinosid, Mansonin, Streblosid, Hydrocortison, Betamethason, Dexamethason, nichtsteroidale Substanzen (NSAIDS), Fenoporfen, Ibuprofen, Indomethacin, Naproxen, Phenylbutazon, antivirale Agentien, Acyclovir, Ganciclovir, Zidovudin, Clotrimazol, Flucytosin, Griseofulvin, Ketoconazol, Miconazol, Nystatin, Terbinafin, antiprozoale Agentien, Chloroquin, Mefloquin, Quinin, natürliche Terpenoide, Hippocaesculin, Barringtogenol-C21-angelat, 14-Dehydroagrostistachin, Agroskerin, Agrostistachin, 17-Hydroxyagrostistachin, Ovatodiolide, 4,7-Oxycycloanisomelsäure, Baccharinoide B1, B2, B3 und B7, Tubeimosid, Bruceantinoside C, Yadanzioside N, und P, Isodeoxyelephantopin, Tomenphantopin A und B, Coronarin A,B,C und D, Ursolsäure, Hyptatsäure A, Iso-Iridogermanal. Maytenfoliol, Effusantin A, Excisanin A und B, Longikaurin B, Sculponeatin C, Kamebaunin, Leukamenin A und B, 13,18-Dehydro-6-alpha-Senecioyloxychaparrin, Taxamairin A und B, Regenilol, Triptolid, Cymarin, Hydroxyanopterin, Protoanemonin, Cheliburinchlorid, Sinococulin A und B, Dihydronitidin, Nitidinchlorid, 12-beta-Hydroxypregnadien 3,20-dion, Helenalin, Indicin, Indicin-N-oxid, Lasiocarpin, Inotodiol, , Podophyllotoxin, Justicidin A und B, Larreatin, Malloterin, Mallotochromanol, Isobutyrylmallotochromanol, Maquirosid A, Marchantin A, Maytansin, Lycoridicin, Margetin, Pancratistatin, Liriodenin, Bispsrthenolidin, Oxoushinsunin, Periplocosid A, Ursolsäure, Deoxypsorospermin, Psycorubin, Ricin A, Sanguinarin, Manwuweizsäure, Methylsorbifolin, Sphatheliachromen, Stizophyllin, Mansonin, Streblosid, Dihydrousambaraensin, Hydroxyusambarin, Strychnopentamin, Strychnophyllin, Usambarin, Usambarensin, Liriodenin, Oxoushinsunin, Daphnoretin, Lariciresinol, Methoxylariciresinol, Syringaresinol, Sirolimus (Rapamycin), Somatostatin, Tacrolimus, Roxithromycin, Troleandomycin, Simvastatin, Rosuvastatin, Vinblastin, Vincristin, Vindesin, Teniposid, Vinorelbin, Tropfosfamid, Treosulfan, Tremozolomid, Thiotepa, Tretinoin, Spiramycin, Umbelliferon, Desacetylvismion A, Vismion A und B, Zeorin.

9. Expandierbare Vorrichtung nach einem der vorherigen Ansprüche, wobei, wenn es sich bei der expandierbaren Vorrichtung um eine Gefäßstütze handelt, die Oberfläche der Gefäßstütze mit einer Carbonschicht versehen ist.

10. Expandierbare Vorrichtung nach einem der vorherigen Ansprüche, wobei, wenn es sich bei der expandierbaren Vorrichtung um eine Gefäßstütze handelt, die Oberfläche der Gefäßstütze mit einer porösen Kohlenstoffschicht versehen ist.

11. Expandierbare Vorrichtung nach einem der vorherigen Ansprüche, wobei die expandierbare Vorrichtung mit einer äußeren hämokompatiblen Schicht versehen ist.

12. Expandierbare Vorrichtung nach einem der vorherigen Ansprüche, wobei die expandierbare Vorrichtung mit einer äußeren Barriereschicht versehen ist.

13. Verfahren zur Beschichtung einer expandierbaren Vorrichtung zur Aufweitung eines Gefäßlumens umfassend die folgenden Schritte:
a) Bereitstellung einer expandierbaren Vorrichtung,
b) Aufbringen von elementarem Iod oder einer Iod-Komplexverbindung oder Aufbringen einer Beschichtung aus elementarem Iod oder einer lod-Komplexverbindung und einem Träger mittels Besprühen, Tauchen, Streichen, Bepinseln, Spritzen oder durch Vakuumabscheidungsverfahren.

14. Verfahren nach Anspruch 13 umfassend die Schritte:
I) Bereitstellen einer Vakuumkammer,
II) Platzierung der expandierbaren Vorrichtung mittels Haltemittel in der Vakuumkammer,
III) Befüllung einer oder mehrerer Kavitäten innerhalb der Vakuumkammer mit einer Iod-Lösung, elementarem Iod als Feststoff, einer Iod-Träger-Lösung oder einer Iod-Lösung und einer Träger-Lösung,
IV) Anlegen von Vakuum an die Vakuumkammer,
V) Erzeugung von Ultraschall in mindestens einer Kavität, welche die zum Aufbringen auf die expandierbare Vorrichtung vorgesehenen Substanzen enthält,
VI) Aufbringen der mittels Ultraschall zerstäubten Substanzen auf die expandierbare Vorrichtung,
VII) Belüftung der Vakuumkammer und Entnahme der beschichteten expandierbaren Vorrichtung.

15. Expandierbare Vorrichtung erhältlich nach dem Verfahren gemäß Patentanspruch 13 oder 14.
